# NEUE EUROPÄISCHE PATENTSCHRIFT

(11) **EP 2 041 281 B2**
(45) Veröffentlichungstag und Bekanntmachung des Hinweises auf die Entscheidung über den Einspruch: **19.02.2014**
(45) Hinweis auf die Patenterteilung: 15.09.2010
(21) Anmeldenummer: 07787162.2
(22) Anmeldetag: 06.07.2007
(51) Int. Cl.: C12N 15/10

(54) **VERFAHREN ZUR PARALLELEN ISOLIERUNG DOPPEL- UND EINZELSTRÄNGIGER NUKLEINSÄUREN SOWIE ZUR SELEKTIVEN ENTFERNUNG DOPPELSTRÄNGIGER NUKLEINSÄUREN AUS EINEM GEMISCH VON DOPPEL- UND EINZELSTRÄNGIGEN NUKLEINSÄUREN**
METHOD FOR INSULATING IN PARALLEL DOUBLE AND SINGLE-STRANDED NUCLEIC ACIDS AND FOR SELECTIVELY REMOVING DOUBLE-STRANDED NUCLEIC ACIDS FROM A MIXTURE OF DOUBLE AND SINGLE-STRANDED NUCLEIC ACIDS
PROCÉDÉ PERMETTANT D'ISOLER EN PARALLÈLE DES ACIDES NUCLÉIQUES À SIMPLE BRIN ET À DOUBLE BRIN ET DE RETIRER DE FAÇON SÉLECTIVE DES ACIDES NUCLÉIQUES À DOUBLE BRIN D'UN MÉLANGE D'ACIDES NUCLÉIQUES À SIMPLE BRIN ET À DOUBLE BRIN

(30) Priorität: 06.07.2006 DE 102006031764
(43) Veröffentlichungstag der Anmeldung: 01.04.2009
(73) Patentinhaber: AJ Innuscreen GmbH, 13125 Berlin (DE)
(72) Erfinder: HILLEBRAND, Timo, D-15366 Hoenow (DE)
(74) Vertreter: Wehlan, Helmut
(86) Internationale Anmeldenummer: PCT/EP2007/056879
(87) Internationale Veröffentlichungsnummer: WO 2008/003776

(56) Entgegenhaltungen:
- EP-A- 1 146 049
- WO-A-97/28171
- WO-A-97/30152
- US-A- 5 155 018
- SCHENK J A ET AL: "FAST ISOLATION OF RNA TO DETECT EXPRESSION OF TUMOR MARKERS" JOURNAL OF CLINICAL LABORATORY ANALYSIS, NEW YORK, NY, US, Bd. 11, 1997, Seiten 340-342, XP000982430 ISSN: 0887-8013
- GRIBANOV ET AL: 'Simple Method for Isolation and Purification of RNA' BIOORGANISCHESKAYA KHIMIYA Bd. 23, Nr. 9, 1977, Seiten 763 - 765

## Beschreibung

Die Erfindung betrifft ein Verfahren zur effizienten und extrem einfachen und kostengünstigen parallelen Isolierung doppelsträngiger und einzelsträngiger Nukleinsäuren. Unter doppelsträngiger Nukleinsäure wird insbesondere genomische DNA und unter einzelsträngiger Nukleinsäure RNA verstanden.

Die parallele Isolierung von genomischer DNA und zellulärer Total RNA aus einer biologischen Probe ist bis zum heutigen Zeitpunkt an nur einige wenige praktizierbare Methoden gebunden. Von Raha, S., Merante, F., Proteau, G. und Reed, J.K. (GATA, 1990, 7(7): 173-177) wird eine Methode zur Trennung genomischer DNA und zellulärer Total RNA über selektive Präzipitationsschritte unter Verwendung von Lithiumchlorid beschrieben. Eine weitere Möglichkeit der parallelen Isolierung von DNA und RNA nutzt Ultrafiltrationstechniken unter Verwendung eines Cäsiumchloridgradienten zur Pelletierung der RNA in Kombination mit nachfolgender Dialyse der DNA (Coombs, L.M., Pigott, D., Proctor, A., Eydmann, M., Denner, J. und Knowles, M.A.; Anal. Biochem. (1990); 188; 338-343). Das publizierte Verfahren ist extrem zeitaufwendig und benötigt einen speziellen apparativen Aufwand.

Nachteilig an diesem Verfahren sind aber die aufwendigen Präparationsschritte und die Verwendung von hochtoxischen und kanzerogenen Substanzgruppen wie Chloroform und Phenol. Letztlich ist auch der notwendige Zeitbedarf sehr groß.

Die Veröffentlichung "SIMPLE METHOD FOR ISOLATION AND PURIFICATION OF RNA" in Bioorganicheskaya Khimiya 23(9):763-765, 1997 von Gribanov et al. beschreibt die Trennung von doppel- und einzelsträngigen Nukleinsäuren durch Zugabe eines chaotropen Salzes, wobei die doppelsträngige Nukleinsäure an einen festen Träger bindet. Die Verwendung eines nichtchaotropen Salzes wird nicht offenbart. Die Anbindung von Nukleinsäuren auch mittels antichaotroper Salze an feste Phasen wird in der Druckschrift WO 00/34463 beschrieben. Dabei wird allerdings keine Trennung von doppel-und einzelsträngigen Nukleinsäuren erreicht.

Ein vereinfachtes Verfahren zur simultanen Isolierung von DNA und RNA ist in der Offenlegungsschrift WO 97/28171 A1 offenbart. Hierbei wird die biologische Probe mit einem chaotropen Puffer lysiert. Nach Lyse erfolgt die Zugabe von Nanopartikeln (kleiner 40nm) aus monodispersem Siliziummaterial. An diese Partikel bindet die genomische DNA. Der Ansatz wird nachfolgend zentrifugiert. Dabei werden die Siliziumpartikel pelletiert. Der Überstand wird in ein neues Reaktionsgefäß überführt. Das pelletierte Trägermaterial wird anschließend mit einem ethanolhaltigen Waschpuffer gewaschen und die gebundene DNA final mit einem Niedrigsalzpuffer wieder vom mineralischen Trägermaterial abgelöst. Der zurückgebliebene Überstand wird dann in klassischer Weise einer Phenol/Chloroform-Extraktion unterzogen und die RNA auf diese Weise letztlich präzipitiert und nach Waschschritten mittels Wasser gelöst.

Das Verfahren ist schneller als die oben beschriebene Methode. Allerdings wird auch in diesem Verfahren mit hochtoxischen und kanzerogenen Substanzgruppen wie Chloroform und Phenol gearbeitet.

Ein weiteres Verfahren der Trennung und Isolierung von einzelsträngigen und doppelsträngigen Nukleinsäuren wird in der Patentschrift EP 1 146 049 B1 offenbart.

Das Verfahren basiert auf der Behandlung einer Nukleinsäuren enthaltenden Quelle mit mindestens einem mineralischen Trägermaterial in der Form dass:
1. die einzelsträngige Nukleinsäure isoliert wird, indem man die Behandlungsbedingungen durch ein wässriges Gemisch von chaotropen Salzen und niederen aliphatischen Alkoholen einstellt, derart, dass nachfolgend vorrangig die einzelsträngige Nukleinsäure an einen mineralischen Träger adsorbiert wird, die doppelsträngige Nukleinsäure dagegen nicht adsorbiert. Die gebundene einzelsträngige Nukleinsäure wird nachfolgend gewaschen und final mittels eines Niedrigsalzpuffers vom Trägermaterial eluiert.
2. die doppelsträngige Nukleinsäure isoliert wird, indem man die Behandlungsbedingungen durch ein Gemisch von Erdalkali-Ionen komplexierenden Substanzen ohne niedere aliphatische Alkohole einstellt, derart , dass nachfolgend vorrangig die doppelsträngige Nukleinsäure an einen mineralischen Träger adsorbiert wird, die einzelsträngige Nukleinsäure dagegen nicht adsorbiert. Die gebundene doppelsträngige Nukleinsäure wird nachfolgend gewaschen und final mittels eines Niedrigsalzpuffers vom Trägermaterial eluiert.
3. die doppelsträngige Nukleinsäure isoliert wird, in dem man die Behandlungsbedingungen durch die Anwesenheit eines Sarkosinats aber ohne niedere aliphatische Alkohole einstellt, derart, dass nachfolgend vorrangig die doppelsträngige Nukleinsäure an einen mineralischen Träger adsorbiert wird, die einzelsträngige Nukleinsäure dagegen nicht adsorbiert. Die gebundene doppelsträngige Nukleinsäure wird nachfolgend gewaschen und final mittels eines Niedrigsalzpuffers vom Trägermaterial eluiert.
4. die doppelsträngige oder einzelsträngige Nukleinsäuren isoliert wird, in dem man die Behandlungsbedingungen durch ein wässriges Gemisch von chaotropen Salzen und niederen aliphatischen Alkoholen einstellt, derart, dass beide Nukleinsäurefraktionen an einen mineralischen Träger adsorbieren. Die Trennung der Nukleinsäuren erfolgt durch eine selektive Elution. Dabei wird die doppelsträngige Nukleinsäure mittels einer Lösung verminderter Ionenstärke und niederen aliphatischen Alkoholen vom Trägermaterial abgelöst. Die verbleibende einzelsträngige Nukleinsäure wird nachfolgend gewaschen und final mittels eines Niedrigsalzpuffers vom Trägermaterial abgelöst.

Alternativ dazu kann die einzelsträngige Nukleinsäure mittels einer Lösung, welche Erdalkali-Ionen komplexierende Substanzen und/oder Sarkosinate enthält, selektiv vom Trägermaterial eluiert werden. Die nunmehr verbleibende doppelsträngige Nukleinsäure wird nachfolgend wiederum gewaschen und final mittels eines Niedrigsalzpuffers vom Trägermaterial abgelöst.

Das Verfahren ist einfach in der Durchführung und verzichtet gänzlich auf die Verwendung von Phenol oder Chloroform. Eine zeitaufwendige Ethanolfällung ist ebenfalls nicht notwendig.

Allerdings zeigt sich auch, dass die Selektivität der Isolierung der jeweiligen Nukleinsäuren oftmals nicht gegeben ist. So benötigt z.B. ein kommerziell angebotener Extraktions-Kit zur Isolierung von RNA auf der Basis eines der beschriebenen Verfahren immer einen DNase Verdau. Dieser ist notwendig, da die dargestellte Selektivität zur Isolierung der RNA allein mit dem beschriebenen Verfahren nicht ausreichend ist. Die doppelsträngige DNA muss zusätzlich mittels eines enzymatischen Verfahrens entfernt werden.

Die Druckschrift WO97/37040 A2 verweist ebenfalls auf ein Verfahren zu Trennung von einzelsträngiger und doppelsträngiger Nukleinsäuren, insbesondere in Hinblick auf die Isolierung von HCV RNA aus Körperflüssigkeiten. Wie schon in der Patentschrift EP 1 146 049 B1 offenbart, erfolgt auch in WO 97/37040 A2 die Anbindung der doppelsträngigen Nukleinsäure an ein Silicamaterial dadurch, dass der dafür eingesetzte Puffer eine hohe Konzentration an EDTA (mindestens 100 mM) benötigt. Damit wird die Anwesenheit eines Komplexbildners in hoher Konzentration benötigt, um doppelsträngige Nukleinsäure an mineralische Silicapartikel zu binden und die Anbindung von einzelsträngiger Nukleinsäure zu verhindern.

Im US-Patent 5,155,018 wird ein Verfahren und ein Testkit zur RNA-Isolierung und Reinigung von biologischen Quellen beschrieben. Darin wird im Beispiel 2 die Isolierung von RNA beschrieben, wobei als Lösung ein Gemisch aus 5M GuanidinThiocyanat, 0.25% Essigsäure and 1% 2-Mercaptoethanol verwendet wird.

Der vorliegenden Erfindung lag die Aufgabe zu Grunde, die Nachteile der bekannten Verfahren zu beseitigen und ein besseres Verfahren zur Trennung einzel- und doppelsträngiger Nukleinsäuren zur Verfügung zu stellen.

Die Aufgabe wurde gemäß den Merkmalen des Patentanspruchs 1 gelöst. Die Patentansprüche 2 bis 9 stellen bevorzugte Ausführungsformen der Erfindung dar.

Das erfindungsgemäße Verfahren zur parallelen Isolierung doppel- und einzelsträngiger Nukleinsäuren sowie zur selektiven Entfernung doppelsträngiger Nukleinsäuren aus einem Gemisch von doppel- und einzelsträngigen Nukleinsäuren oder aus diese Stoffe enthaltenden Quellen, ist durch folgende Schritte gekennzeichnet:
a) die lysierte oder homogenisierte Probe wird in Abwesenheit von Alkohol oder Erdalkali-Ionen komplexierende Substanzen oder Wasch-, Dispensier oder Netzmitteln mit einer wässrigen **<nichtchaotropen>** Salzlösung in einer Konzentration größer 1M so eingestellt, dass die doppelsträngige Nukleinsäure an einen festen Träger adsorbiert wird, während die einzelsträngige Nukleinsäure nicht adsorbiert wird und in der Lösung bleibt
b) der Trägers mit der adsorbierten Nukleinsäure wird entfernt
c) die Lösung mit der einzelsträngigen Nukleinsäure wird mit einer alkoholischen Lösung in einer Konzentration von 1 bis 90 Vol.-% versetzt und mit einem weiteren festen Träger in Kontakt gebracht, wobei die einzelsträngige Nukleinsäure an diesen weiteren Träger adsorbiert wird.

Die an den jeweiligen festen Träger adsorbierte Nukleinsäure wird nach bekannten Methoden gewaschen und eluiert. Als fester Träger dient vorzugsweise ein mineralischer Träger. Der feste Träger kann Bestandteil einer Mini-Zentrifugationssäule sein.

Für die Adsorption der doppelsträngigen Nukleinsäuren wird gemäß einer bevorzugten Ausführungsform das gleiche Trägermaterial wie für die Adsorption der einzelsträngigen Nukleinsäuren verwendet.

Als alkoholische Lösung werden vorzugsweise Alkohole mit 1 bis 5 Kohlenstoffatomen eingesetzt. Die alkoholische Lösung kann zusätzlich weitere Stoffe, wie Natrium- oder Kaliumacetatpuffer oder ein Alkohol-Detergenzgemisch, insbesondere nichtionische Detergenzien enthalten.

Für die Absorption der einzelsträngigen Nukleinsäure kann als fester Träger ein mineralischer Träger oder magnetische Eisenoxidpartikel, vorzugsweise mit modifizierten Oberflächen, eingesetzt werden.

Ein Kit zur Durchfiihrung des Verfahrens ist aus EP 1 146049 A2 (Absatz [0041] in Kombination mit den Absätzen [0038] bis [0040]) prinzipiell bekannt und umfasst folgende Bestandteile::
- eine wässrige Salz-Lösung mit einer Salz-Konzentration größer 1M
- zwei feste Träger
- eine alkoholische Lösung in einer Konzentration von 1 bis 90 Vol.-%
- bekannte Wasch- und Elutionspuffer.

Bei der wässrigen Salzlösung handelt es sich um eine Lösung von nichtkomplexbildenden Salzen.

Beschrieben wird somit auch die Verwendung einer wässrigen **<nichtchaotropen>** Salzlösung mit einer Salz-Konzentration größer 1M zur selektiven Entfernung doppelsträngiger Nukleinsäuren aus einem Gemisch von doppel- und einzelsträngigen Nukleinsäuren oder aus diese Stoffe enthaltenden Quellen durch Bindung der doppelsträngigen Nukleinsäuren an einen festen Träger.

Beschrieben wird auch die Verwendung einer wässrigen **<nichtchaotropen>** Salzlösung mit einer Salz-Konzentration größer 1M zur Isolierung hochreiner zellulärer Total-RNA, die frei von genomischer DNA ist, aus einem Gemisch von zellulärer Total-RNA und genomischer DNA oder aus diese Stoffe enthaltenden Quellen durch Bindung der genomischen DNA an einen festen Träger.

Erfindungsgemäß wurde ein Verfahren bereitgestellt, welches es ermöglicht, parallel doppel- und einzelsträngige Nukleinsäuren aus einer Nukleinsäuren enthaltenden Quelle zu isolieren, wobei das Verfahren einfach und schnell in der Durchführung ist und auf die Verwendung von Phenol oder Chloroform gänzlich verzichtet.

Das erfindungsgemäße Verfahren ermöglicht es, doppel- und einzelsträngige Nukleinsäuren aus einer Nukleinsäuren enthaltenden Quelle zu trennen, mit dem Ziel der selektiven Isolierung nur der einzelsträngigen Nukleinsäure, wobei das Verfahren wiederum einfach und schnell in der Durchführung ist und auf die Verwendung von Phenol oder Chloroform gänzlich verzichtet.

Es zeigte sich, dass man eine Trennung von einzelsträngiger und doppelsträngiger Nukleinsäure auf einem völlig anderen Weg, als in der Patentschrift EP 1 146 049 B1 beschrieben, erreichen kann. In EP 1 146 049 B1 sind zwei Verfahrensalternativen zur Anbindung von doppelsträngiger Nukleinsäure an einen festen Träger genannt worden, nämlich:
1. Puffer, welche ein Gemisch von Erdalkali-Ionen komplexierende Substanzen enthalten, also z.B. EDTA oder
2. Puffer, welche ein Netz,- Wasch oder Dispergiermittel (Sarkosinate) enthalten

In EP 1 146 049 B1 bewirken gerade diese Bedingungen - nach Inkubation mit einem mineralischen Trägermaterial - eine selektive Anbindung der doppelsträngigen Nukleinsäure, wobei die einzelsträngige Nukleinsäure nicht bindet. Die gebundene doppelsträngige Nukleinsäure wird nachfolgend ggf. gewaschen und final mittels Niedrigsalzpuffer oder Wasser vom mineralischen Trägermaterial eluiert. Die nichtgebundene einzelsträngige Nukleinsäure kann dann nachfolgend ebenfalls isoliert werden, indem zum Beispiel die Bindungsbedingungen durch Zugabe eines Alkohols so einstellt werden, dass eine effiziente Adsorption an ein weiteres mineralisches Material erfolgt. Auch die gebundene einzelsträngige Nukleinsäure wird nachfolgend gewaschen und final wiederum vom mineralischen Material abgelöst.

Weiterhin wird in EP 1 146 049 B 1 erwähnt, dass bei Zugabe eines wässrigen Gemischs von chaotropen Salzen in einer Konzentration größer 1M und niederen aliphatischen Alkoholen die Behandlungsbedingungen so eingestellt werden können, dass überwiegend die einzelsträngige Nukleinsäure oder aber die Gesamtnukleinsäure an den festen Träger adsorbiert wird.

Ein entscheidender Unterschied der vorliegenden Erfindung gegenüber EP 1 146 049 B 1 ist also, dass die wässrige **<nichtchaotrope>** Salzlösung in einer Konzentration größer 1M - in Abwesenheit von Alkohol - bewirkt, dass nicht (wie in EP 1 146 049 B1) die einzelsträngige Nukleinsäure oder Gesamtnukleinsäure, sondern ausschließlich die doppelsträngige Nukleinsäure an den festen Träger adsorbiert wird. Dieses Ergebnis war überraschend und nicht aus dem Stand der Technik ableitbar.

Auch in WO 97/37040 A2 werden hohe Salzkonzentrationen komplexierender Komponenten (EDTA; EGTA) für die Anbindung einer doppelsträngigen Nukleinsäure benötigt.

Damit weisen zwei unterschiedliche Patentschriften, welche die selbe Zielstellung beschreiben, explizit darauf hin, dass für die Anbindung einer doppelsträngigen Nukleinsäure ein komplexierende Komponenten (EDTA; EGTA) die wesentliche Komponente darstellt.

Die vorliegende Erfindung zeigt aber überraschenderweise gerade den gegenteiligen Effekt, nämlich, dass die Anbindung von doppelsträngiger Nukleinsäure gerade dann hervorragend realisiert wird, wenn kein komplexierendes Agens vorliegt. Dieser Effekt war damit in keiner Weise zu erwarten.

Es hat sich demnach herausgestellt, dass im erfindungsgemäßen Verfahren die Anbindung von doppelsträngiger Nukleinsäure an ein festes Trägermaterial unter genau den entgegengesetzten Bindungsbedingungen erfolgt. Die Adsorption der doppelsträngigen Nukleinsäuren erfolgt dann, wenn die o.g. **<nichtchaotrope>** Salzlösung keinen Alkohol enthält. In diesem Fall kann auch zur Adsorption der doppelsträngigen Nukleinsäuren auf Erdalkali-Ionen komplexierende Substanzen oder Sarkosinate oder andere Wasch-, Dispergier-oder Netzmittel verzichtet werden.

Das Verfahren zur parallelen Isolierung von genomischer DNA und zellulärer RNA realisiert sich mittels des erfindungsgemäßen Verfahrens damit wie folgt:

Die Nukleinsäure enthaltende Quelle wird ggf. homogenisiert oder lysiert, wobei der Lysepuffer eine hohe **<nichtchaotrope>** Salzkonzentration (> 1M), aber im Gegensatz zur Patentschrift EP 1146049 keinen Alkohol, keine Erdalkali-Ionen komplexierende Substanzen und/oder keine Wasch-, Dispensier- oder Netzmittel enthält. Nach Homogenisierung/Lyse wird die aufgeschlossene Probe mit einem festen Träger, vorzugsweise ein mineralischen Trägermaterial, in Kontakt gebracht. Das Trägermaterial kann dabei in unterschiedlichster Form zugeführt werden. In einer bevorzugten Ausführungsvariante ist das Trägermaterial Bestandteil einer Mini- Zentrifugationssäule.

Unter den angegebenen Bedingungen bindet die genomische DNA (doppelsträngige Nukleinsäure) an das Trägermaterial, nicht aber die RNA (einzelsträngige Nukleinsäure). Das Filtrat enthält die RNA und wird für den weiteren Extraktionsprozess aufbewahrt. Die am Trägermaterial gebundene genomische DNA wird anschließend ggf. mit einem alkoholischen Waschpuffer gewaschen und final mittels eines Niedrigsalzpuffers oder Wasser vom Trägermaterial eluiert. Das aufbewahrte und die einzelsträngige Nukleinsäure enthaltende Filtrat wird mit einer alkoholischen Lösung versetzt und nunmehr ebenfalls mit einem weiteren mineralischen Trägermaterial in Kontakt gebracht. Das mineralische Material kann wiederum in unterschiedlichster Form vorliegen, vorzugsweise wieder als Bestandteil einer Zentrifugationssäule. Dabei kann das Trägermaterial für die Anbindung der RNA aus dem selben mineralischen Material wie für die Anbindung der genomischen DNA sein.

Zur Erhöhung der Effizienz der Anbindung der RNA kann die alkoholische Lösung ggf. noch weitere Salze, insbesondere Natriumacetat- oder Kaliumacetatpuffer mit sauren pH-Werten oder ein Alkohol-Detergenzgemisch, insbesondere nichtionische Detergenzien (z.B. Tween20, Tween80, TritonX-100), enthalten.

Nach erfolgter Bindung der RNA an das eingesetzte Trägermaterial erfolgen ggf. an sich bekannte Waschschritte. Final wird auch die RNA mittels eines Niedrigsalzpuffers oder mittels Wasser vom Trägermaterial eluiert.

Mittels dieser extrem einfachen Verfahrensabläufe kann schnell und effizient DNA und RNA aus einer biologischen Probe isoliert werden.

In einer weiteren erfindungsgemäßen Ausführungsalternative kann mittels der Trennung von doppelsträngiger und einzelsträngiger Nukleinsäure auch hocheffizient und schnell hochreine zelluläre Total RNA, welche weitestgehend frei von kontaminierender genomischer DNA ist, isoliert werden, so dass die bisher Verwendung findenden enzymatischen DNA-Verdauschritte nicht mehr notwendig sind.

Hierzu nutzt man den beschriebenen erfindungsgemäßen Mechanismus der selektiven Adsorption von doppelsträngiger Nukleinsäure (DNA) an ein mineralisches Trägermaterial, wobei aber mit der gebundenen DNA nicht mehr weitergearbeitet wird. Nach der Entfernung der genomischen DNA aus der Ausgangsprobe wird diese wie oben beschrieben nachfolgend mit einer alkoholischen Lösung, welche ggf. weitere Salzkomponenten enthalten kann, gemischt und dieser Ansatz mit einem weiteren mineralischen Trägermaterial kontaktiert, das Trägermaterial mit der gebundenen RNA ggf. mit einem alkoholischen Waschpuffer gewaschen und final die RNA mittels eines Niedrigsalzpuffers bzw. mittels Wasser vom Trägermaterial eluiert. Das Verfahren zur selektiven Isolierung hochreiner RNA, frei von genomischer DNA, besticht durch seine Schnelligkeit und Einfachheit.

Interessanterweise können für die spezifische Anbindung der beiden Nukleinsäurefraktionen dieselben mineralischen Materialien eingesetzt werden. Es besteht aber auch die Möglichkeit, unterschiedliche Materialien für die selektive Anbindung von DNA und RNA einzusetzen. So kann z.B. die selektive Entfernung der genomischen DNA schnell und effizient unter Verwendung einer Zentrifugationssäule mit einem Glasfasermaterial erfolgen, wobei nachfolgend die Anbindung der RNA nach Zugabe der beschriebenen alkoholischen Komponente an magnetische Eisenoxidpartikel, vorzugsweise mit modifizierten Oberflächen, erfolgt. Hierbei kombiniert man dann z.B. einen manuellen Schritt zur Entfernung von DNA aus einer biologischen Probe mit einem automatisierten Verfahren (z.B. automatisierte Magnetpartikelseparation) mit dem Ziel, schnell hochreine RNA zu isolieren.

Die mittels der erfindungsgemäßen Verfahrensvarianten isolierten Nukleinsäuren (genomische DNA und/ oder RNA) sind undegradiert und von exzellenter Qualität (OD₂₆₀:OD₂₈₀= 1.8-2.0). Die Kontamination mit genomischer DNA ist vernachlässigbar und dies ohne die bisher benötigten enzymatischen Schritte (Verwendung von DNAse I).

Die Verfahren erlauben sowohl die parallele Isolierung von DNA und RNA als auch die selektive Isolierung von RNA aus prinzipiell jeder biologischen Ausgangsprobe. Dabei können auch extrem geringe Mengen an Ausgangsmaterial einer Isolierung zugänglich gemacht werden. Dies ist insbesondere bei der Bearbeitung von mikrodissektierten Proben wesentlich. Gerade bei diesen extrem limitierten Probenmengen ist eine parallele Isolierung von DNA und RNA von bedeutendem Vorteil.

Die Erfindung wird nachfolgend an einem Ausführungsbeispiel näher erläutert, wobei das Ausführungsbeispiel keine Limitierung des erfindungsgemäßen Verfahrens darstellt.

### Ausführungsbeispiel

### Parallele Isolierung genomischer DNA und zellulärer Total RNA aus einer Gewebeprobe

Überführen von 20 mg Lebergewebe der Maus in ein 1.5 ml Reaktionsgefäß. Zugabe von 450 µl Lysepuffer (4 M Guanidinthiocyanat, 80 mM tri-Natriumcitrat-Dihydrat). Zerreiben der Gewebe Probe mittels eines Mikro-Homogenisators. Inkubation für 30 min bei Raumtemperatur. Überführen des Lyseansatzes auf eine Filtersäule mit Auffanggefäß (Glasfaserfilter Fa. Whatmann) und Zentrifugation für 2 min bei 10.000 x g. Bei diesem Schritt bindet die genomische DNA an die Oberfläche der Filtersäule. Die Filtersäule mit der gebundenen DNA wird in ein neues Auffanggefäß gesteckt. Das erhaltenen Filtrat enthält die zelluläre Total RNA und wird mit einem gleichen Volumen an 70%igem Ethanol versetzt. Dieser Ansatz wird dann auch auf eine Filtersäule (Glasfaserfilter Fa. Whatmann) mit Auffanggefäß überführt und ebenfalls bei 10.000 x g für 1 min zentrifugiert. Das Filtrat wird verworfen und die Filtersäule mit der gebundenen RNA wird in ein neues Auffanggefäß gesteckt. Die beiden Filtersäulen werden dann mit einem ethanolhaltigen Waschpuffer (z.B. 80% Ethanol, 50 mM NaCl, 10 mM Tris HCl) gewaschen und nachfolgend mittels Zentrifugation getrocknet. Die Elution der gebunden genomischen DNA erfolgt mittels der Zugabe von 100 µl Elutionspuffer (10 mM Tris HCl) oder Wasser. Die Elution der zellulären Total RNA erfolgt mittels der Zugabe von 100 µl RNase freiem Wassers. Beide Filtersäulen werden für 1 min bei 10.000 x g zentrifugiert. Die Filtersäulen werden verworfen, die Filtrate enthalten die beiden Nukleinsäurefraktionen.

## Patentansprüche

1. Verfahren zur parallelen Isolierung doppel- und einzelsträngiger Nukleinsäuren sowie zur selektiven Entfernung doppelsträngiger Nukleinsäuren aus einem Gemisch von doppel-und einzelsträngigen Nukleinsäuren oder aus diese Stoffe enthaltenden Quellen, **gekennzeichnet durch** folgende Schritte:
a) die lysierte oder homogenisierte Probe wird in Abwesenheit von Alkohol, Erdalkali-Ionen komplexierenden Substanzen und Wasch-, Dispensier- oder Netzmitteln mit einer wässrigen nichtchaotropen Salzlösung in einer Konzentration größer 1M so eingestellt, dass die doppelsträngige Nukleinsäure an einen festen Träger adsorbiert wird, während die einzelsträngige Nukleinsäure nicht adsorbiert wird und in der Lösung bleibt
b) Entfernen des Trägers mit der adsorbierten Nukleinsäure
c) die Lösung mit der einzelsträngigen Nukleinsäure wird mit einer alkoholischen Lösung in einer Konzentration von 1 bis 90 Vol.-% versetzt und mit einem weiteren festen Träger in Kontakt gebracht, wobei die einzelsträngige Nukleinsäure an diesen weiteren Träger adsorbiert wird.

2. Verfahren nach Anspruch 1, **dadurch gekennzeichnet, dass** die an den jeweiligen festen Träger adsorbierte Nukleinsäure nach bekannten Methoden gewaschen und eluiert wird.

3. Verfahren nach einem der Ansprüche 1 oder 2, **dadurch gekennzeichnet, dass** als fester Träger ein mineralischer Träger verwendet wird.

4. Verfahren nach einem der Ansprüche 1 bis 3, **dadurch gekennzeichnet, dass** der feste Träger Bestandteil einer Mini-Zentrifugationssäule ist.

5. Verfahren nach einem der Ansprüche 1 bis 4, **dadurch gekennzeichnet, dass** für die Adsorption der doppelsträngigen Nukleinsäuren das gleiche Trägermaterial wie für die Adsorption der einzelsträngigen Nukleinsäuren verwendet wird.

6. Verfahren nach einem der Ansprüche 1 bis 5, **dadurch gekennzeichnet, dass** die alkoholische Lösung Alkohole mit 1 bis 5 Kohlenstoffatomen enthält.

7. Verfahren nach Anspruch 6, **dadurch gekennzeichnet, dass** die alkoholische Lösung zusätzlich weitere Stoffe, wie
a) Natrium- oder Kaliumacetatpuffer oder
b) ein Alkohol-Detergenzgemisch, insbesondere nichtionische Detergenzien enthält.

8. Verfahren nach einem der Ansprüche 1 bis 5, **dadurch gekennzeichnet, dass** für die Absorption der einzelsträngigen Nukleinsäure als fester Träger ein mineralischer Träger oder magnetische Eisenoxidpartikel, vorzugsweise mit modifizierten Oberflächen, eingesetzt werden.

## Claims

1. A method for parallel isolation of double-stranded and single-stranded nucleic acids as well as for selective removal of double-stranded nucleic acids from a mixture of double-stranded and single-stranded nucleic acids or from sources containing these substances, **characterised by** the following steps :
a) the lysed or homogenised sample, in the absence of alcohol, substances complexing alkaline earth ions and detergents, dispensing or wetting agents, by means of an aqueous, non-chaotropic, saline solution of a concentration greater than 1M, is adjusted such that the double-stranded nucleic acid is adsorbed to a solid support, whereas the single-stranded nucleic acid is not adsorbed and remains in the solution
b) removal of the support with the adsorbed nucleic acid
c) an alcoholic solution with a concentration of 1 to 90 percent by volume is added to the solution with the single-stranded nucleic acid and placed in contact with another solid support with the single-stranded nucleic acid being adsorbed to this other support.

2. A method according to claim 1, **characterised in that** the nucleic acid adsorbed to the respective solid carrier is washed and eluted in accordance with known methods.

3. A method according to any one of claims 1 or 3, **characterised in that** a mineral support is used as a solid support.

4. A method according to any one of claims 1 to 3, **characterised in that** the solid support is an integral part of a mini spin column.

5. A method according to any one of claims 1 to 4, **characterised in that** for adsorption of the double-stranded nucleic acids the same support material is used as for adsorption of the single-stranded nucleic acids.

6. A method according to any one of claims 1 to 5, **characterised in that** the alcoholic solution contains alcohol with 1 to 5 carbon atoms.

7. A method according to claim 6, **characterised in that** the alcoholic solution contains further substances in addition such as
a) sodium or potassium acetate buffers or
b) an alcohol-detergent mixture, in particular non-ionic detergents.

8. A method according to any one of claims 1 to 5, **characterised in that** for adsorption of the single-stranded nucleic acids a mineral support or magnetic iron oxide particles, preferably with modified surfaces, are used as a solid support.

## Revendications

1. Procédé pour l'isolation parallèle des acides nucléiques bicaténaires et monocaténaires ainsi que pour l'élimination sélective des acides nucléiques bicaténaires à partir d'un mélange des acides nucléiques bicaténaires et monocaténaires ou à partir des sources contenant ces substances, **caractérisé par** les étapes suivantes :
a) l'échantillon lysée ou homogénéisée en absence de l'alcool ou des substances complexant des ions alcalinoterreux et des agents de lavage, de dispensation ou des mouillants avec une solution saline aqueuse non chaotrope dans une concentration plus grande que 1M est ajustée de manière que l'acide nucléique bicaténaire est adsorbé à un porteur solide tandis que l'acide nucléique monocaténaire n'est pas adsorbé et reste dans la solution
b) élimination du porteur avec l'acide nucléique adsorbé
c) une solution alcoolique d'une concentration de 1 à 90 pourcent volumétrique est additionnée à la solution avec l'acide nucléique monocaténaire et est amenée en contact avec un autre porteur solide, l'acide nucléique monocaténaire étant adsorbé à cet autre porteur.

2. Procédé selon la revendication 1, **caractérisé en ce que** l'acide nucléique adsorbé au porteur solide respectif est lavé et élué selon des méthodes connues.

3. Procédé selon l'une quelconque des revendications 1 ou 2, **caractérisé en ce qu'**un porteur minéral est utilisé en tant que porteur solide.

4. Procédé selon l'une quelconque des revendications 1 à 3, **caractérisé en ce que** le porteur solide est une partie constituante d'une mini-colonne de centrifugation.

5. Procédé selon l'une quelconque des revendications 1 à 4, **caractérisé en ce que** pour l'adsorption des acides nucléiques bicaténaires le même matériau porteur est utilisé que pour l'adsorption des acides nucléiques monocaténaires.

6. Procédé selon l'une quelconque des revendications 1 à 5, **caractérisé en ce que** la solution alcoolique comprend des alcools avec 1 à 5 atomes de carbone.

7. Procédé selon la revendication 6, **caractérisé en ce que** la solution alcoolique comprend en outre d'autres substances telles que
a) des tampons acétate de sodium ou des tampons acétate de potassium ou
b) un mélange alcool-détergent, surtout des détergents non ioniques.

8. Procédé selon l'une quelconque des revendications 1 à 5, **caractérisé en ce que** pour l'adsorption de l'acide nucléique monocaténaire un porteur minéral ou des particules magnétiques d'oxyde de fer, de préférence avec des surfaces modifiées, sont utilisés en tant que porteur solide.
